# EUROPÄISCHE PATENTSCHRIFT

(11) **EP 0 438 756 B1**
(45) Veröffentlichungstag und Bekanntmachung des Hinweises auf die Patenterteilung: **20.04.1994**
(21) Anmeldenummer: 90125093.6
(22) Anmeldetag: 21.12.1990
(51) Int. Cl.: A61K 37/02

(54) **Cytostatische und cytotoxische Wirkstoffkombination zur Anwendung in therapeutischen Verfahren**
Combination of cytostatic and cytotoxic active agents for use in therapeutic procedures
Combinaisons d'agents actifs cytostatiques et cytotoxiques pour applications dans des méthodes thérapeutiques

(30) Priorität: 04.01.1990 DE 4000154
(43) Veröffentlichungstag der Anmeldung: 31.07.1991
(73) Patentinhaber: SYMBIOTEC Gesellschaft zur Forschung und Entwicklung auf dem Gebiet der Biotechnologie GmbH, D-35745 Herborn (DE)
(72) Erfinder: Zeppezauer, Michael, Prof. Dr., W-6601 Scheidt (DE); Leinenbach, Hans-Peter, Dr., W-6601 Riegsberg (DE)
(74) Vertreter: Pätzold, Herbert, Dr.-Ing.

(56) Entgegenhaltungen:
- EP-A- 0 316 030
- DE-A- 3 737 274
- DATABASE BIOSIS, Accession Nr. 89006585; S.H. KAUFMAN: "Induction ofendonucleolytic DNA cleavage in human acute myelogenous leukemia cells by etoposide camptothecin and other cytotoxic anticancer drugs, a cautionary note"
- DATABASE CANCERLIT, Accession Nr. 77000655; W. CREASEY: "Basic mechanisms oftissue injury occasioned by chemotherapy"

## Beschreibung

Die Erfindung bezieht sich auf ein Arzneimittel aus einem ersten und einem zweiten Wirkstoff, die am Ort des pathogenen Prozesses eine synergistische Wirkung entfalten.

In der Krebstherapie werden Cytostatika verwendet. Die frühe Diagnose und die rechtzeitige Entfernung von Tumoren sind bis heute der Schlüssel für eine erfolgreiche Krebstherapie. Die Strahlentherapie wird bei lokal begrenzten Tumoren und als additive Therapie zur Chirurgie ebenfalls mit Erfolg eingesetzt. Bei disseminierenden und metastasierenden Tumoren bleiben nur noch die Chemo- und Immunotherapie als Behandlungsmethode. Die Chemotherapien führen stets schwere Nebenwirkungen mit sich. So verursachen die im Kombinationsversuch verwendeten Cytostatika Nierenschäden, Leberschäden, Störung der Hämatopoese und viele andere Schäden, die das Allgemeinbefinden und die Widerstandskraft des Patienten stark beeinträchtigen. Dazu kommt, daß fast alle Cytostatika stark immunosuppressiv wirken. Diese Nebenwirkungen bedingen, daß die Dosierung der Cytostatika oft nicht hoch genug gewählt werden kann, oder daß die Therapie vor Einsetzen eines Erfolges abgebrochen werden muß. Erschwerend kommt hinzu, daß bei längerem Einsatz von Cytostatika oft eine Resistenz der Krebszellen auftritt. Schließlich gibt es Krebszellen, gegen die die bekannten Cytostatika wirkungslos sind.

Immunotherapien werden hauptsächlich mit Interferonen und Interleukinen zur Stimulierung der zellulären Abwehr durchgeführt. In der Regel handelt es sich hierbei nur um unterstützende additive Maßnahmen.

Wegen ihrer immunosuppressiven Wirkung werden Cytostatika auch zur Therapie von Autoimmunerkrankungen, wie z.B. bei Rheuma, Multiple Sclerose und Psoriasis angewendet, wobei auch hier schwere Nebenwirkungen in Kauf genommen werden müssen, weswegen auch hier die Dosierung oftmals nicht ausreichend hoch genug angesetzt werden kann oder vorzeitig abgebrochen werden muß.

Aufgabe der vorliegenden Erfindung ist es, eine Wirkstoffkombination bereitzustellen, die eine wesentlich verbesserte cytostatische oder sogar cytotoxische Wirkung als die herkömmlichen Cytostatika, z.B. Vincristin, Methotrexat, Cisplatin allein aufweisen, um durch Senkung der wirksamen Dosen des Cytostatikums effektivere Chemotherapien als bisher mit wesentlich gemilderten oder weitgehend verringerten Nebenwirkungen durchführen zu können. Durch die Wirkstoffkombination soll die therapeutische Wirksamkeit von bekannten Cytostatika auch erhöht oder überhaupt erst ermöglicht werden.

In der EP-A- 0 316 030 ist zur Krebstherapie die Wirkstoffkombination aus Vincristin und einem stark basischen Protein offenbart, das basische Aminosäuren enthält. Die angegebene Wirkung soll durch die Wirkstoffkombination von Vincristin mit der speziellen synthetischen Sequenz des basischen Proteins erzielt werden, wobei die hier enthaltenden Aminosäuren für die Wirkung aber nicht ausschlaggebend sind.

Durch die EP-Patentanmeldung 85 100 179.2 ist bereits bekanntgeworden, daß wenigstens ein Histon und/oder wenigstens ein Histonabschnitt hormonelle Wirkungen aufweisen kann, die sich vorteilhaft z.B. zur Krebstherapie anwenden lassen. Insbesondere handelt es sich um die Histone H1, H2A und/oder H2B und H3.

In der deutschen Offenlegungsschrift 37 37 274 ist die direkte cytotoxische Wirkung von H2A/H2B-Histongemisch auf bestimmte Krebszellinien gezeigt. Es ist die Verwendung von reinem H2A- und/oder H2B-Histon als Wirksubstanz mit hormonellen bzw. hormonähnlichen Eigenschaften offenbart.

In der US-A-4,818,763 ist die cytotoxische Wirkung von H1 auf verschiedene Krebszellinien offenbart.

Die Aufgabe wird für ein Arzneimittel der eingangs genannten Art erfindungsgemäß dadurch gelöst, daß der erste Wirkstoff wenigstens ein Cytostatika ist und daß der zweite Wirkstoff mit cytostatischer oder cytotoxischer Wirksamkeit wenigstens ein Histon oder wenigstens ein Histonabschnitt (aktiver Histonabschnitt) oder eine Kombination aus wenigstens einem Histon und wenigstens einem aktiven Histonabschnitt ist.

Vorteilhafte Ausführungen nach der Erfindung ergeben sich aus den Merkmalen der Unteransprüche. Hiernach ist es vorteilhaft, wenn der zweite Wirkstoff aus der Gruppe H1, H2A, H2B, H2A:H2B und H3 ausgewählt ist. Es kann auch vorteilhaft sein, wenn bei dem zweiten Wirkstoff der aktive Histonabschnitt der evolutionär variable Abschnitt ist. Weiterhin kann es von Vorteil sein, wenn bei dem zweiten Wirkstoff der aktive Histonabschnitt der N- oder C-terminale Abschnitt ist. Auch kann es vorteilhaft sein, wenn es sich bei dem zweiten Wirkstoff um wenigstens einen Histonabschnitt des H2A mit der Teilsequenz 1-41 oder 1-36 oder 1-12 oder 1-11 oder 12-36 oder 12-41 oder 1-28 oder 11-23 handelt. Vorteile lassen sich auch dadurch erzielen, wenn es sich bei dem zweiten Wirkstoff um wenigstens einen Histonabschnitt des Histonuntertyps H2A Z mit der Teilsequenz 1-30 oder 9-25 handelt.

Die erfindungsgemäße Wirkstoffkombination kann auch darin bestehen, daß es sich bei dem zweiten Wirkstoff um wenigstens einen Histonabschnitt des Histons H2B mit der Teilsequenz 1-35 oder 1-34 oder 1-32 oder 1-31 oder 1-29 oder 1-26 oder 1-23 oder 24-35 oder 24-34 oder 24-32 oder 24-31 oder 24-29 oder 1-20 oder 21-29 oder 21-31 oder 21-32 oder 21-34 oder 21-35 oder 14-26 handelt. Bei dem zweiten Wirkstoff kann es sich aber auch um einen Histonabschnitt des Histonuntertyps H2B P.A. mit der Teilsequenz 17-30 handeln. Vorteilhaft kann es auch sein, wenn es sich bei dem zweiten Wirkstoff um einen Histonabschnitt des Histons H3 mit der Teilsequenz 3-34 und 17-29 handelt. Die Erfindung kann aber auch darin bestehen, daß es sich bei dem zweiten Wirkstoff um wenigstens ein Histon und/oder einen Histonabschnitt handelt, das bzw. der wenigstens eine der repetitiven Aminosäuresequenzen ausgewählt aus der Gruppe KRAA (= Lys Arg Ala Ala) und KRVA (= Lys Arg Val Ala) aufweist. Hierbei kann es auch vorteilhaft sein, wenn es sich bei dem Wirkstoff um einen C-terminalen Abschnitt von H1 oder einem Teil hiervon mit der repetitiven Sequenz der Aminosäuren KRAA (= Lys Arg Ala Ala) und/oder um einen N-terminalen Abschnitt von H2B oder einem Teil hiervon mit der repetitiven Sequenz der Amniosäuren KRVA (= Lys Arg Val Ala) handelt, wobei es vorteilhaft sein kann, wenn der erste Wirkstoff ausgewählt ist aus der Gruppe Vincristin, Methatrexat und Cisplatin und wenn der zweite Wirkstoff H2A:H2B ist.

Das erfindungsgemäße Arzneimittel kann vorteilhafterweise zur Krebstherapie und zur Therapie von Autoimmunkrankheiten dienen.

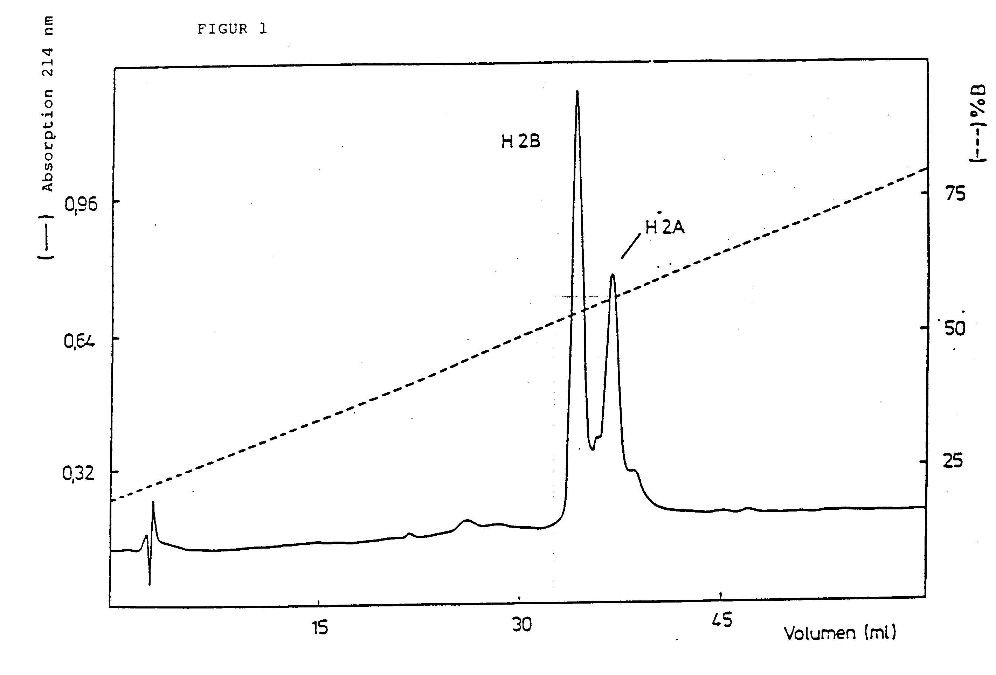

Die Wirksamkeit der erfindungsgemäßen Wirkstoffkombination wird anhand der nachstehenden Versuche in Verbindung mit Diagrammen für Ausführungsbeispiele aufgezeigt, die in der anliegenden Zeichnung mit den Fig. 1 bis 7 bezeichnet sind.

Es wird jeweils ein H2A:H2B-Histongemisch bzw. - ein H2A:H2B-Histonkomplex entsprechend Fig. 1 verwendet, das bzw. der aus einer Thymus-Präparation (homöostatische Thymushormon) aus Kalbsthymus nach Comsa & Bernardi ("Extraction, Fractionation and Testing of the Homogenous Thymic Hormone Preparation, Annals of the New York Academy of Sciences, Vol. 240, S. 402-403; 28.02.1975) mittels Hochleistungsflüssigkeits-Chromatographie gewonnen wurde. Die Elution erfolgte an einer µBondpak C-18-Säule mit einem linearen Gradienten (%B) aus 20 bis 80% Acetonitril in 0,1 % Trifluoressigsäure bei einer Flußrate von 1 ml/min. Die Absorption wurde bei 214 nm gemessen. In Fig. 1 ist der Abszisse das Volumen in ml, auf der linken Ordinate die Absorption bei 214 nm und auf der rechten Ordinate der lineare Gradient (%B) aufgetragen.

Entsprechend läßt sich reines H2A und reines H2B herstellen. Es wird noch zu klären sein, ob H2A:H2B (Fig. 1) ein Gemisch aus H2A und H2B oder eine Verbindung aus H2A und H2B (H2A:H2B-Komplex) ist. Es ist klar, daß auch andere bekannte Verfahren zur Darstellung der Histone verwendet werden können. Die Erfindung ist daher nicht auf die Verwendung von H2A:H2B beschränkt, sondern erstreckt sich auch auf deren aktiven Teile mit cytostatischer oder cytotoxischer Wirkung.

Obwohl der cytostatische oder cytotoxische Wirkstoffmechanismus von Histonen oder deren aktiven Abschnitten noch nicht restlos aufgeklärt ist, spricht erfinderseits einiges dafür, daß den repetitiven Aminosäuresequenzen KRAA (= Lys Arg Ala Ala) und KRVA (= Lys Arg Val Ala) eine aktive Bedeutung zukommt. Die erstere befindet sich z.B. im C-terminalen Abschnitt von H1 und die letztere ist z.B. im N-terminalen Abschnitt von H2B vorhanden.

Die malignen Zellen wurden mit komplettiertem Kulturmedium (RPMI 1640 mit 10 % FCS) gefüttert. Das Kulturmedium wurde täglich erneuert. Nach Zuwachsen des Kulturschalenbodens wurden die Zellen abgeschabt und teilweise umgesetzt, da für die Versuche Zellen im optimalen Wachstumsbereich benötigt wurden. Die Bebrütung erfolgte bei 36,5°C und 5,5% Co₂ in einem Brutschrank.

Die Lebendzellkonzentration wurde mit dem Farbstoff Nigrosin (0,2 % in phosphatgepufferter Kochsalzlösung (PBS) in der Neubauer-Zählkammer bestimmt.

Bestandteile des erfindungsgemäßen Chemotherapeutika (z.B. H2A:H2B und ein bekanntes Cytostatika) wurden jeweils einzeln als auch in erfindungsgemäßer Kombination mit dem Kulturmedium versetzt und steril filtriert.

Für die Versuche wurden die malignen Zellen vom Boden von nicht zu dicht bewachsenen Kulturschalen abgeschabt, die Lebendzellzahl bestimmt und auf 3,5 x 10⁵ Zellen/ml eingestellt. Ein Teil dieser Zellsuspension wurden mit dein erfindungsgemäßen Chemotherapeutikum bzw. mit dessen Einzelbestandteilen versetzt und im Brutschrank inkubiert. Die endkonzentration der malignen Zellen pro Napf betrug 1,75 x 10⁵ Zellen/ml.

### Versuch 1

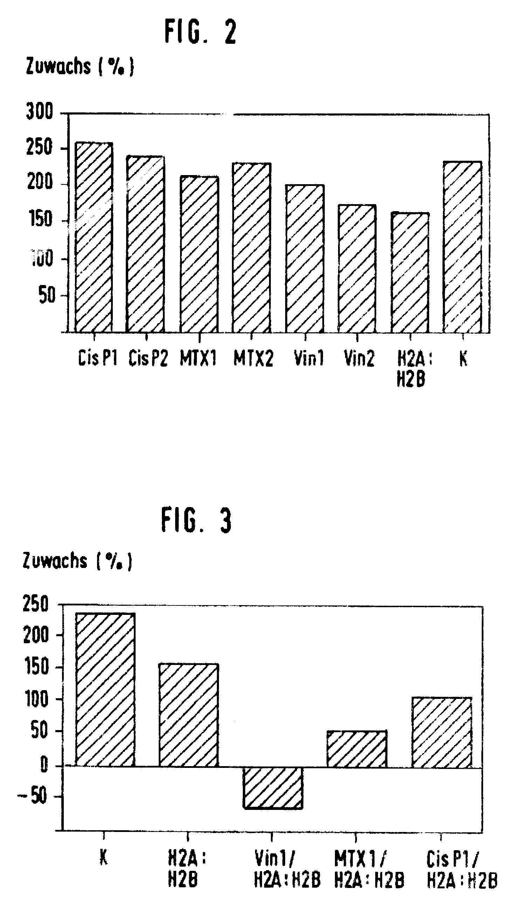

Die Wirksamkeit von H2A:H2B in Kombination mit den Cytostatika Cisplatin, Methotrexat und Vincristin wurde in vitro an der OH 77 Lymphomzellinie getestet. Fig. 2 zeigt einen Cytotoxizitätstest mit den vorstehenden Cytostatika und H2A:H2B jeweils für sich gegenüber Zellen der OH 77 Lymphomzellinie. Hierzu wurden Zellen dieser Zellinie für 48 h mit CisP1 (1 µg/ml Cisplatin), CisP2 (2µg/ml Cisplatin , MTX1 (5 µg/ml Methotrexat), MTX2 (10 µg/ml Methotrexat), Vin1 (5 µg/ml Vincristin), Vin2 (10 µg/ml Vincristin) und 250 µg/ml H2A:H2B inkubiert und jeweils der Zellzuwachs in % notiert. Mit K ist die Zellwachstumskontrolle über 48 h (Zellwachstum ohne Zusatz eines Cytostatika oder H2A:H2B) bezeichnet. Die Cytostatika und das H2A:H2B zeigen nur geringe oder gar keine cytostatische Wirkungen.

Fig. 3 zeigt einen Cytotoxizitätstest, wobei die vorstehenden Cytostatika jeweils einzeln mit H2A:H2B kombiniert angewendet sind. Im einzelnen wurden die Zellen der OH 77 Lymphomzellinie für 48 h mit Vin1/H2A:H2B (5 µg/ml Vincristin + 100 µg/ml H2A:H2B), MTX1/H2A:H2B (5 µg/ml Methotrexat + 100 µg/ml H2A:H2B) und CisP1/H2A:H2B (1 µg/ml Cisplatin + 100 µg/ml H2A:H2B) inkubiert und der Zellwachstum in % notiert. Mit K ist wiederum die Zellwachstumskontrolle über 48h ohne jeden Zusatz bezeichnet, und zum besseren Vergleich mit H2A:H2B ist die cytostatische Wirkung allein von 100 µg/ml H2H:H2B gezeigt.

Die Kombination von H2A:H2B + Vincristin jeweils in Konzentrationen, die für die einzelnen Substanzen nur eine leicht cytostatische Wirkung zeigte (Fig. 2), führte dagegen zu einer synergistischen Wirkung mit cytotoxischem Effekt (Fig. 3).

Die cytostatischen Wirkungen mit den Kombinationen H2A:H2B + Methotrexat bzw. H2A:H2B + Cisplatin sind gegenüber den einzelnen Stoffen verbessert. Das gilt vor allem für die Kombination H2A:H2B + Methotrexat.

### Versuch 2

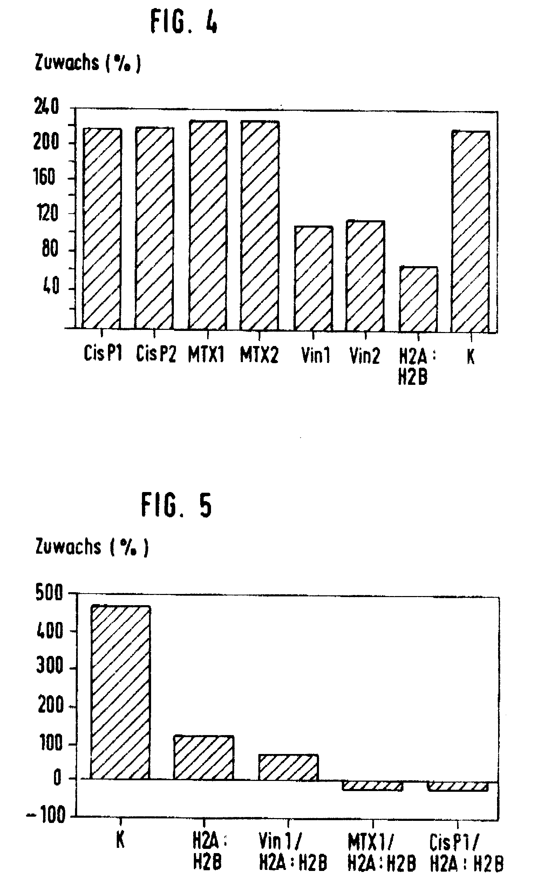

Die Wirksamkeit von H2A:H2B in Kombination mit den genannten Cytostatika wurde in vitro an der EG 463 Melanomzellinie getestet. Fig. 4 zeigt einen weiteren Cytotoxizitätstest mit den betreffenden Cytostatika und H2A:H2B jeweils für sich gegenüber Zellen der EG 463 Melanomzellinie. Zellen dieser Zellinie wurden für 48 h mit folgenden Substanzen jeweils einzeln inkubiert und der Zellwachstum in % notiert: CisP1 (1 µg/ml Cisplatin), CisP2 (2 µg/ml Cisplatin), MTX1 (5 µg/ml Methotrexat), MTX2 (10 µg/ml Methotrexat), Vin1 (5 5 µg/ml Vincristin), Vin2 (10 µg/ml Vincristin) und 250 µg/ml H2A:H2B. Mit K ist wiederum die Zellwachstumskontrolle über 48 h ohne jeden Zusatz bezeichnet.

In Fig. 5 wurden die Zellen der EG 463 Melanomzellinie für 48 h mit den vorstehenden Cytostatika in Kombination mit H2A:H2B in folgender Weise inkubiert und der Zellwachstum in % notiert:
Vin1/H2A:H2B (5 µg/ml Vincristin + 100 µg/ml H2A:H2B), MTX1/H2A:H2B (5 µg/ml Methotrexat + 100 µg/ml H2A:H2B, CisP1/H2A:H2B (1 µg/ml Cisplatin + 100 µg/ml H2A:H2B), Mit K ist wiederum die Zellwachstumskontrolle über 48 h ohne jeden Zusatz bezeichnet und H2A:H2B verdeutlicht die cytostatische Wirkung allein von 100 µg/ml H2A:H2B. Fig. 5 verdeutlicht, daß die Kombination von H2A:H2B + Vincristin, die beide als einzelne Substanzen leicht cytostatisch wirken (Fig. 4), nicht synergistisch, sondern nur additiv wirksam sind.

Dagegen zeigt Methotrexat und Cisplatin, die beide als einzelne Substanzen nicht einmal cytostatisch wirksam sind (Fig. 4), jeweils in Kombination mit H2A:H2B cytotoxische (synergistische)Wirkung (Fig. 5).

### Versuch 3

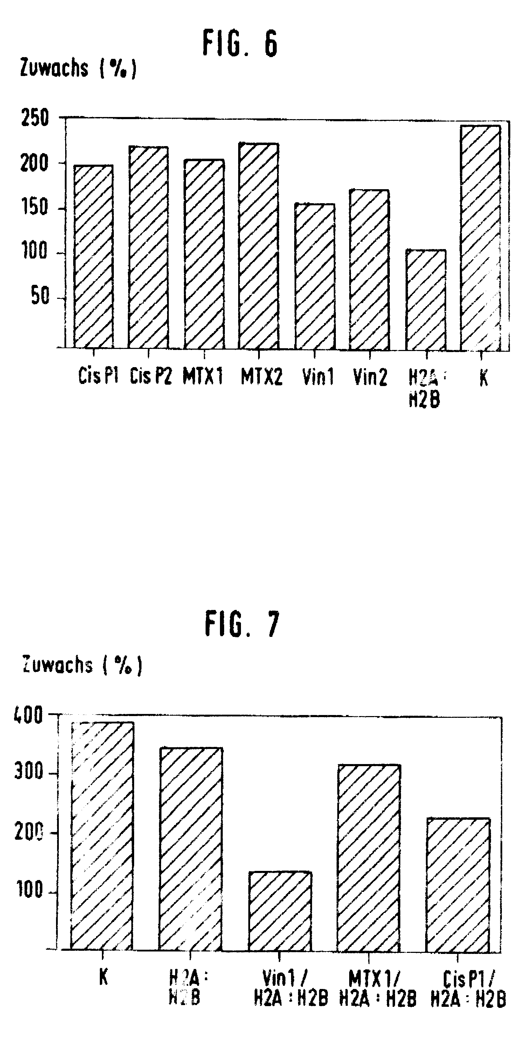

Die Wirksamkeit von H2A:H2B in Kombination mit den genannten Cytostatika wurde in vitro auch an der Hufibl Fibrolastenzellinie getestet. Fig. 6 zeigt einen noch weiteren Cytotoxizitätstest mit den Cytostatika und H2A:H2B jeweils für sich gegenüber der Hufibl Fibroblastenzelllinie. Zellen dieser Zellinie wurden für 48 h mit folgenden einzelnen Substanzen inkubiert und der Zellwachstum in % notiert.

CisP1 (1 µg/ml Cisplatin), CisP2 (2 µg/ml Cisplatin), MTX1 (5 µg/ml Methotrexat), MTX2 (10 µg/ml Methotrexat), Vin1 (5 µg/ml Vincristin), Vin2 (10 µg/ml Vincristin) und 250 µg/ml H2A:H2B. Mit K ist erneut die Zellwachstumskontrolle über 48 h ohne jeden Zusatz bezeichnet.

In Fig. 7 werden die Zellen der Hufibl Fibroblastenzellinie für 48 h mit den vorstehenden Cytostatika in Kombination mit H2A:H2B in der folgenden Weise inkubiert und der Zellwachstum in % notiert:
Vin1/H2A:H2B (5 µg/ml Vincristin + 100 µg/ml H2A:H2B), MTX1/H2A:H2B (5 µg/ml Methotrexat + 100 µg/ml H2A:H2B) und CisP1/H2A:H2B (1 µg/ml Cisplatin + 100 µg/ml H2A:H2B).

Außerdem wurde die Zellinie zum Vergleich nur mit 100 µg/ml H2A:H2B inkubiert. Mit K ist wiederum die Zellwachstumskontrolle ohne jeden Zusatz bezeichnet.

Eine synergistische Wirkung von H2A:H2B mit Cytostatika ist bei den untransformierten humanen Fibroblasten nicht zu beobachten. Die bereits vorhandene cytostatische Wirkung der Chemotherapeutika wird durch H2A:H2B erhöht (Fig. 6), aber nicht in eine cytotoxische Wirkung umgekehrt.

Die Erfindung ist nicht auf die Kombination von H2A:H2B-Histongemisch bzw. -Histonkomplex mit Cytostatika beschränkt. Es ist zu vermuten, daß vergleichbare Wirkungen auch allein mit dem H2A-Histon oder mit dem H2B-Histon in Kombination mit Cytostatika erzielt werden. Weiterhin ist zu vermuten, daß vergleichbare Wirkungen auch die Histone H1 und H3 in Kombination mit Cytostatika aufweisen. Im übrigen ist dem Fachmann klar, daß statt der Histone auch nur deren aktive Bestandteile genommen werden können, die aus mindestens vier oder fünf Aminosäureresten bestehen und cytostatische oder cytotoxische Eigenschaften zeigen.

Die Erfindung ist schließlich nicht auf die genannten Cytostatika beschränkt. Nach der Lehre der Erfindung kann der Fachmann beliebige andere Cytostatika mit wenigstens einem Histon oder aktiven Histonabschnitt in Kombination bringen, um nach der erfinderischen Lehre neue kombinierte Chemotherapeutika bereitzustellen, die wenigstens eine erhöhte cytostatische Wirkung trotz verringerter Dosierung der verwendet Cytostatika aufweisen, so daß selbst bei erhöhter cytostatischer Wirkung die Nebenwirkungen der Cytostatika wesentlich gemildert werden können, oder es können neue kombinierte Chemotherapeutika angeboten werden, die sogar eine synergistische Wirkung mit cytotoxischem Effekt aufweisen, wobei auch hier die Dosierung der Cytostatika gesenkt und entsprechend die Nebenwirkungen gemildert werden können. Durch die synergistische Wirkung können bisher unwirksame Cytostatika erst ihre Wirksamkeit erzielen, die für sich allein keine therapeutische Wirksamkeit bei der Behandlung von Krebserkrankungen oder Autoimmunerkrankungen aufweisen.

Da die Verabreichung der Histone keine Nebenwirkungen aufweisen, besteht nach der Lehre der Erfindung nunmehr die Möglichkeit in Kombination mit Cytostatika die Chemotherapie mit wesentlich erhöhten Erfolgsaussichten über einen längeren Zeitraum durchführen zu können, wobei die Nebenwirkungen der Cytostatika in erträglichen Grenzen gehalten werden können.

Die erfindungsgemäße Wirkstoffkombination kann so verabreicht werden, daß der erste und der zweite Wirkstoff gemeinsam oder getrennt verabreicht werden. Die synergistische Wirkung der erfindungsgemäßen Wirkstoffkombination tritt jeweils am Ort des pathogenen Prozesses auf, unabhängig davon, ob die einzelnen Wirkstoffe gemeinsam oder getrennt verabreicht werden.

Obwohl die vorstehend angegebenen Versuche nur die Wirksamkeit der erfindungsgemäßen Wirkstoffkombination gegen Krebszellen, insbesondere Lymphdrüsenkrebs und malignen Melanom zeigen, ist die Erfindung nicht auf die Anwendung in therapeutischen Verfahren zur Behandlung von Krebserkrankungen beschränkt. Es liegt nahe, die erfindungsgemäße Wirkstoffkombination auch in therapeutischen Verfahren zur Behandlung von Autoimmunerkrankungen anzuwenden.

## Patentansprüche

1. Arzneimittel aus einem ersten und einem zweiten Wirkstoff, die am Ort des pathogenen Prozesses eine synergistische Wirkung entfalten,
dadurch gekennzeichnet, daß der erste Wirkstoff wenigstens ein Cytostatika ist und daß der zweite Wirkstoff mit cytostatischer oder cytotoxischer Wirksamkeit wenigstens ein Histon oder wenigstens ein Histonabschnitt (aktiver Histonabschnitt) oder eine Kombination aus wenigstens einem Histon und wenigstens einem aktiven Histonabschnitt ist.

2. Wirkstoffkombination nach Anspruch 1,
dadurch gekennzeichnet,
daß der zweite Wirkstoff aus der Gruppe H1, H2A, H2B, H2A:H2B und H3 ausgewählt ist.

3. Wirkstoffkombination nach Anspruch 1,
dadurch gekennzeichnet,
daß bei dem zweiten Wirkstoff der aktive Histonabschnitt der evolutionär variable Abschnitt ist.

4. Wirkstoffkombination nach Anspruch 1,
dadurch gekennzeichnet,
daß bei dem zweiten Wirkstoff der aktive Histonabschnitt der N- oder C-terminale Abschnitt ist.

5. Wirkstoffkombination nach Anspruch 1,
dadurch gekennzeichnet,
daß es sich bei dem zweiten Wirkstoff um wenigstens einen Histonabschnitt des H2A mit der Teilsequenz 1-41 oder 1-36 oder 1-12 oder 1-11 oder 12-36 oder 12-41 oder 1-28 oder 11-23 handelt

6. Wirkstoffkombination nach Anspruch 1,
dadurch gekennzeichnet,
daß es sich bei dem zweiten Wirkstoff um wenigstens einen Histonabschnitt des Histonuntertyps H2A Z mit der Teilsequenz 1-30 oder 9-25 handelt.

7. Wirkstoffkombination nach Anspruch 1,
dadurch gekennzeichnet,
daS es sich bei dem zweiten Wirkstoff um wenigstens einen Histonabschnitt des Histons H2B mit der Teilsequenz 1-35 oder 1-34 oder 1-32 oder 1-31 oder 1-29 oder 1-26 oder 1-23 oder 24-35 oder 24-34 oder 24-32 oder 24-31 oder 24-29 oder 1-20 oder 21-29 oder 21-31 oder 21-32 oder 21-34 oder 21-35 oder 14-26 handelt.

8. Wirkstoffkombination nach Anspruch 1,
dadurch gekennzeichnet
daß es sich bei dem zweiten Wirkstoff um einen Histonabschnitt des Histonuntertyps H2B P.A. mit der Teilsequenz 17-50 handelt.

9. Wirkstoffkombination nach Anspruch 1,
dadurch gekennzeichnet,
daß es sich bei dem zweiten Wirkstoff um einen Histonabschnitt des Histons H3 mit der Teilsequenz 3-34 und 17-29 handelt.

10. Wirkstoffkombination nach Anspruch 1,
dadurch gekennzeichnet,
daß es sich bei dem zweiten Wirkstoff um wenigstens ein Histon und/oder einen Histonabschnitt handelt, das bzw. der wenigstens eine der repetitiven Aminosäuresequenzen ausgewählt unter der Gruppe KRAA (Lys Arg Ala Ala) und KRVA (Lys Arg Val Ala) aufweist.

11. Wirkstoffkombination nach Anspruch 10, dadurch gekennzeichnet, daß es sich bei dem Wirkstoff um einen C-terminalen Abschnitt von H1 oder einem Teil hiervon mit der repetitiven Sequenz der Aminosäuren KRAA (Lys Arg Ala Ala) und/oder um einen N-terminalen Abschnitt von H2B oder einem Teil hiervon mit der repetitiven Sequenz der Aminosäuren KRVA (Lys Arg Val Ala) handelt.

12. Wirkstoffkombination nach Anspruch 10 oder 11,
dadurch gekennzeichnet,
daß der erste Wirkstoff ausgewählt ist aus der Gruppe Vincristin, Methotrexat und Cisplatin.

13. Wirkstoffkombination nach Anspruch 12,
dadurch gekennzeichnet,
daß der zweite Wirkstoff H2A:H2B ist.

14. Wirkstoffkombination nach einen der vorstehenden Ansprüche zur Krebstherapie und zur Therapie von Autoimmunerkrankungen.

## Claims

1. Drug comprising a first and a second active agents, which develop a synergistic effect at the locus of the pathogenic process, characterised in that the first active agent is at least a cytostatic, and the second active agent, being cytotoxically or cytostatically effective, is at least a histone or at least a histone part (active histone part), or a combination of at least one histone and at least one active histone part.

2. Combination of active agents according to claim 1, characterised in that the second active agent is selected from the group H1, H2A, H2B, H2A:H2B and H3.

3. Combination of active agents according to claim 1, characterised in that in the second active agent the active histone part is the evolutionarily variable part.

4. Combination of active agents according to claim 1, characterised in that in the second active agent the active histone part is the N- or C-terminal part.

5. Combination of active agents according to Claim 1, characterised in that the second active agent is at least one histone part of H2A with the part sequence 1-41 or 1-36 or 1-12 or 1-11 or 12-36 or 12-41 or 1-28 or 11-23.

6. Combination of active agents according to claim 1, characterised in that the second active agent is at least one histone part of the histone sub-group H2A Z with the part sequence 1-30 or 9-25.

7. Combination of active agents according to claim 1, characterised in that the second active agent is at least one histone part with the part sequence 1-35 or 1-34 or 1-32 or 1-31 or 1-29 or 1-26 or 1-23 or 24-35 or 24-34 or 24-32 or 24-31 or 24-29 or 1-20 or 21-29 or 21-31 or 21-32 or 21-34 or 21-35 or 14-26.

8. Combination of active agents according to claim 1, characterised in that the second active agent is at least one histone part of the histone subtype H2B P.A. with the part sequence 17-50.

9. Combination of active agents according to claim 1, characterised in that the second active agent is at least one histone part of the histone H3 with the part sequence 3-34 and 17-29.

10. Combination of active agents according to claim 1, characterised in that the second active agent is at least one histone and/or a histone part which has at least one of the repetitive aminoacid sequences selected from the group KRAA (Lys Arg Ala Ala) and KRVA (Lys Arg Val Ala).

11. Combination of active agents according to claim 10, characterised in that the active agent is a C-terminal part of H1 or a portion thereof with the repetitive sequence of the aminoacids KRAA (Lys Arg Ala Ala), and/or is an N-terminal part of H2B or a portion thereof with the repetitive sequence of the amino acids KRVA (Lys Arg Val Ala).

12. Combination of active agents according to Claim 10 or 11, characterised in that the first active agent is selected from the group vincristin, methotrexate and cisplatin.

13. Combination of active agents according to claim 12, characterised in that the second active agent is H2A:H2B.

14. Combination of active agents according to one of the preceding claims for cancer therapy and for therapy of autoimmune diseases.

## Revendications

1. Médicament composé d'un premier agent et d'un deuxième agent qui déploient un effet synergique sur le lieu du processus pathogène, caractérisé en ce que le premier agent est au moins un produit cytostatique et en ce que le deuxième agent avec efficacité cytostatique ou cytotoxique est au moins une histone ou un tronçon d'histone (tronçon d'histone actif) ou une combinaison composée d'au moins une histone active et d'au moins un tronçon d'histone actif.

2. Combinaison d'agents selon la revendication 1, caractérisée en ce que le deuxième agent est choisi dans le groupe H1, H2A, H2B, H2A:H2B et H3.

3. Combinaison d'agents selon la revendication 1, caractérisée en ce que, pour le deuxième agent, le tronçon d'histone actif est le tronçon à évolution variable.

4. Combinaison d'agents selon la revendication 1, caractérisée en ce que, pour le deuxième agent, le tronçon d'histone actif est le tronçon terminal N ou C.

5. Combinaison d'agents selon la revendication 1, caractérisée en ce que, pour le deuxième agent, il s'agit au moins d'un tronçon d'histone du H2A avec la séquence partielle 1-41 ou 1-36 ou 1-12 ou 1-11 ou 12-36 ou 12-41 ou 1-28 ou 11-23

6. Combinaison d'agents selon la revendication 1, caractérisée en ce que, pour le deuxième agent, il s'agit d'au moins un tronçon d'histone du sous-modèle d'histone H2A Z avec la séquence partielle 1-30 ou 9-25.

7. Combinaison d'agents selon la revendication 1, caractérisée en ce que, pour le deuxième agent, il s'agit d'au moins un tronçon de la histone H2B avec la séquence partielle 1-35 ou 1-34 ou 1-32 ou 1-31 ou 1-29 ou 1-26 ou 1-23 ou 24-35 ou 24-34 ou 24-32 ou 24-31 ou 24-29 ou 1-20 ou 21-29 ou 21-31 ou 21-31 ou 21-32 ou 21-34 ou 21-35 ou 14-26.

8. Combinaison d'agents selon la revendication 1, caractérisée en ce que, pour le deuxième agent, il s'agit d'un tronçon d'histone du sous-modèle d'histone H2B P.A. avec la séquence partielle 17-50.

9. Combinaison d'agents selon la revendication 1, caractérisée en ce que, pour le deuxième agent, il s'agit d'un tronçon d'histone H3 avec la séquence partielle 3-34 et 17-29.

10. Combinaison d'agents selon la revendication 1, caractérisée en ce que, pour le deuxième agent, il s'agit d'au moins une histone et/ou d'un tronçon d'histone qui présente au moins une des séquences répétitives d'aminoacide choisie dans le groupe KRAA (Lys Arg Ala Ala) et KRVA (Lys Arg Val Ala).

11. Combinaison d'agents selon la revendication 1, caractérisée en ce que, pour le deuxième agent, il s'agit d'un tronçon terminal C de H1 ou d'une partie de celui-ci avec la séquence répétitive des aminoacides KRAA (Lys Arg Ala Ala) et/ou d'un tronçon terminal N de H2B ou d'une partie de celui-ci avec la séquence répétitive des aminoacides KRVA (Lys Arg Val Ala).

12. Combinaison d'agents selon la revendication 1, caractérisée en ce que le premier agent est choisi dans le groupe Vincristine, méthotrexate et cisplatine.

13. Combinaison d'agents selon la revendication 1, caractérisée en ce que le deuxième agent est H2A:H2B.

14. Combinaison d'agents selon l'une des revendications antérieures pour la thérapie anti-cancéreuse et pour la thérapie des maladies autoimmunes.
